# EUROPEAN PATENT SPECIFICATION

(11) **EP 3 630 031 B1**
(45) Date of publication and mention of the grant of the patent: **08.09.2021**
(21) Application number: 18731611.2
(22) Date of filing: 21.05.2018
(51) Int. Cl.: A61F 13/02, A61F 13/00

(54) **ELASTICALLY DEFORMABLE WOUND DRESSINGS**
ELASTISCH VERFORMBARE WUNDVERBÄNDE
PANSEMENTS DÉFORMABLES ÉLASTIQUEMENT

(30) Priority: 22.05.2017 US 201762509607 P
(43) Date of publication of application: 08.04.2020
(73) Proprietor: KCI USA, Inc., San Antonio, TX 78265 (US); KCI Licensing, Inc., San Antonio, TX 78265 (US); Systagenix Wound Management, Limited, Beehive Ring Road, Gatwick Airport, West Sussex RH6 0PA (GB)
(72) Inventor: PIGG, William, Elvington YO41 4DW (GB); LOCKE, Christopher B., Bournemouth BH9 3SD (GB); WAITE, Alexander, Cowling Keighley BD22 0FN (GB); PRATT, Benjamin A., Poole Dorset BH15 3PU (GB); TURTON, Kyle, Skipton YO414DW (GB); HILL, Clinton, Colne Lancashire BB8 9BZ (GB)
(74) Representative: Simmons & Simmons
(86) International application number: PCT/US2018/033637
(87) International publication number: WO 2018/217621

(56) References cited:
- EP-A1- 2 773 799
- WO-A1-2015/145117
- DE-U1-202013 103 953
- US-A1- 2009 216 168
- US-A1- 2013 296 818

## Description

### TECHNICAL FIELD

The subject matter set forth in the appended claims relates generally to treating tissue, including composite dressings that are elastically deformable under treatment conditions.

### BACKGROUND

A wide variety of materials and devices, generally characterized as "wound dressings," are generally known in the art for use in treating an injury or other disruption of tissue. Such wounds may be the result of trauma, surgery, or disease, and may affect skin or other tissues. In general, wound dressings may control bleeding, absorb wound exudate, ease pain, assist in debriding the wound, protect wound tissue from infection, or otherwise promote healing and protect the wound from further damage.

US 2009/0216168 concerns multi-layer wound dressings containing a carrier layer, an absorbing layer, and a hydrophilic wound contact layer connected to the absorbing layer, wherein the wound contact layer comprises a hydrophilic polyurethane elastomer. DE20 2013 103 953 U1 concerns a flat wound care article having a substantially polygonal or elliptical base surface and at least one recess arranged on one side.

Although the clinical benefits and advantages of wound dressings may be widely accepted, improvements to wound dressings may benefit healthcare providers and patients.

### BRIEF SUMMARY

New and useful compositions of dressing layers and dressings including such a dressing layer, methods for manufacturing same, and methods for using the same are set forth in the appended claims. Illustrative embodiments are also provided to enable a person skilled in the art to make and use the claimed subject matter.

Some examples may comprise a composite island dressing, which may comprise an elastic layer, such as a polyurethane or foam layer, coupled to an absorbent layer comprising gelling fibers such as carboxymethyl cellulose fibers. In some examples, the absorbent layer may comprise non-woven fibers. The elastic layer, such as a hydrophilic polyurethane foam layer, may be substantially elastically deformable under tissue treatment conditions, while the absorbent layer may be not elastically deformable under tissue treatment conditions. Nevertheless, the elastic layer and the absorbent layer, when coupled together to form the composite island, may be substantially elastically deformable under tissue treatment conditions.

Some examples may comprise dressings including an absorbent layer comprising gelling cellulosic fibers, for example gelling cellulose ether fibers such as carboxymethyl cellulose fibers, and a backing layer coupled to the absorbent layer. In some examples, the gelling cellulosic fibers may be non-woven. The elastic layer, such as a polyurethane or hydrocolloid backing layer, may be substantially elastically deformable under tissue treatment conditions, while the absorbent layer may be not elastically deformable under tissue treatment conditions. Nevertheless, the elastic layer and the absorbent layer, when coupled together to form the dressing, may be substantially elastically deformable under tissue treatment conditions.

Another example relates to a method of eliminating, minimizing, or reducing edema for a tissue site. The method may comprise positioning a dressing described herein near the tissue site, such that at least a portion of the adherent layer of the dressing contacts the tissue site.

Objectives, advantages, and one or more preferred modes of making and using the claimed subject matter may be understood by reference to the accompanying drawings, in conjunction with the following detailed description of illustrative embodiments.

### DRAWINGS

Figure 1 is a perspective view in cross-section of a dressing according to this specification.
Figure 2 is a diagram of the dressing of Figure 1 with a therapy system.
Figures 3A, 4A, and 5A are perspective top views of three examples of elastic layers of a dressing according to this specification.
Figures 3B, 4B, and 5B are perspective exploded views of three dressings containing the exemplary elastic layers of Figures 3A, 4A, and 5A, respectively.
Figure 6 is a diagram of an electrospinning process for depositing gelling fibers onto a substrate.
Figure 7A is a perspective top view of an example of a dressing according to this specification.
Figures 7B is a perspective angled view of an example result of using a mask in an electrospinning process according to Figure 6 over a portion of a substrate margin when depositing gelling fibers onto a central portion of the substrate.
Figure 7C is a perspective angled view of the dressing from Figure 7B with the mask removed.

It should be noted that the figures set forth herein are intended to exemplify the general characteristics of materials and methods among those of the present invention, for the purpose of the description of certain embodiments. The figures may not precisely reflect all the characteristics of any given embodiment, and are not necessarily intended to define or limit specific embodiments within the scope of this invention.

### DESCRIPTION OF EXAMPLE EMBODIMENTS

The following description of example embodiments provides information that enables a person skilled in the art to make and use the subject matter set forth in the appended claims, but may omit certain details already well-known in the art. The following detailed description is, therefore, to be taken as illustrative and not limiting.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

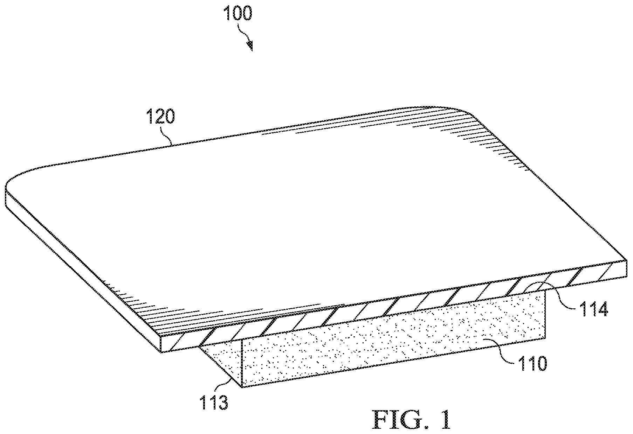

Disclosed herein are embodiments of a dressing layer, embodiments of composite dressings including such a dressing layer, and embodiments of therapy systems including same. Also disclosed herein are embodiments of related methods, such as methods of making and methods of using the disclosed dressing layers, composite dressings, and therapy systems. For example, Figure 1 illustrates an embodiment of a dressing 100. Generally, and as will be disclosed herein, the dressing 100 may be configured to provide therapy to a tissue site in accordance with the disclosure of this specification.

As used herein, "tissue site" is intended to broadly refer to a wound, defect, or other treatment target located on or within tissue, including but not limited to, bone tissue, adipose tissue, muscle tissue, neural tissue, dermal tissue, vascular tissue, connective tissue, cartilage, tendons, or ligaments. A wound may include chronic, acute, traumatic, subacute, and dehisced wounds, partial-thickness burns, ulcers (such as diabetic, pressure, or venous insufficiency ulcers), skin flaps, and grafts, for example. The term "tissue site" may also refer to areas of any tissue that are not necessarily wounded or defective, but are instead areas in which it may be desirable to add or promote the growth of additional tissue, such as granulation. As noted above, the tissue site can refer to an area of tissue where prophylactic treatment is desired, even without a wound or defect, for example with respect to pressure ulcer prevention.

In some embodiments, a dressing may include one or more dressing layers configured to interface with the tissue site. For example, in the embodiment of Figure 1, the dressing 100 may include a dressing layer 110. The dressing layer 110 may generally be configured to be positioned on, over, in, adjacent to, or otherwise in contact with (collectively, "near") the tissue site.

### Dressing Layer

In various embodiments, the dressing layer 110 may be configured so as to be in contact with a portion of the tissue site, substantially all of the tissue site, or the tissue site in its entirety. If the tissue site is a wound, for example, the dressing layer 110 may partially or completely fill the wound, or may be placed over or near the wound. In various embodiments, the dressing layer 110 may take many forms, and may have many sizes, shapes, or thicknesses depending on a variety of factors, such as the type of treatment being implemented or the nature and size of a tissue site. For example, the size and shape of the dressing layer 110 may be adapted to the contours of deep and irregular shaped tissue sites, may be configured so as to be adaptable to a given shape or contour, or both. Moreover, in some embodiments, any or all of the surfaces of the dressing layer 110 may comprise projections or an uneven, course, or jagged profile that can, for example, induce strains and stresses on a tissue site, which may be effective to promote granulation at the tissue site.

In some embodiments, the dressing layer 110 may be in substantially sheet form. For example, the dressing layer 110 may comprise a generally planar structure having two opposite-facing planar surfaces and a depth or thickness orthogonal to the planar surfaces. More particularly, for example, the dressing layer 110 may comprise a first surface 113 opposite a second surface 114, The first surface 113 may be adapted to contact a tissue site, having a surface area sufficient to cover an appropriate portion, if not all, of the tissue site. For example, a surface area from about 1 cm² to about 4000 cm² may be suitable for many applications. In various embodiments, the first surface 113 and the second surface 114 may have any suitable shape, examples of which include, but are not limited to, triangles, squares, rhombuses, rhomboids, diamonds, rectangles, trapezoids, ellipses, ellipsoids, circles, semi-circles, pie-wedges, ovals, and various polygons having four, five, six, seven, eight, or more sides. These shapes may additionally or alternatively be adaptations of such common shapes. In some embodiments, shapes with typically rounded edges may be altered to be flatter, such as a rounded hexagonal/octagonal shape made by flattening the rounded edges of a circle. Additionally or alternatively, shapes with typically rounded edges may be altered to be sharper, such as a tear-drop shape made by sharpening a rounded end of an ellipse or ellipsoid, or such as an eye shape made by sharpening two rounded, opposing ends of an ellipse or ellipsoid. Further additionally or alternatively, shapes with typically pointed edges may be altered to be more rounded, such as for a blunt-ended triangle. Still further additionally or alternatively, shapes with typically flat edges may be altered to be more rounded, such as by converting the flat sides of any regular polygon to a sinusoidal edge to form a doily shape with an undulating, curvy edge. The shape and area of the back surface 114 may be customized to the location and type of tissue site onto which the dressing 100 is to he applied.

### Dressing Layer Composition

There can be various embodiments of the composition of the dressing layer. In some embodiments, the dressing layer 110 may be a single layer, whereas, in some embodiments, the dressing layer 110 may represent a multi-layer composite structure. For example, the dressing layer 110 may comprise at least two layers coupled to each other.

The at least two layers of the multi-layer composite may be coupled to each other using any appropriate technique. In some embodiments, a lamination process can be used to couple layers together, particularly where neither of the layers to be coupled are fibrous. In some embodiments, an adhesive can be used to directly or indirectly couple layers together. In such embodiments, direct adhesion can be used particularly where neither of the layers to be coupled are fibrous, and indirect adhesion can be used particularly where one of the two layers is fibrous and the other of the two layers is not fibrous.

In some embodiments where at least one of the layers is fibrous, a needling apparatus, such as a needle loom machine, can be used to couple the fibrous layer to the other layer, whether fibrous or porous. Needling apparatuses may be used to knit to different types of fibrous materials such as non-wovens together. Typically, in needling apparatuses, a bottom layer can be coupled to a top layer by co-feeding the two layers through the apparatus to be simultaneously needled. By operation of such apparatuses, one or more barbed needles can be punctured into/through the fibrous material(s). Differences in strength or connectivity in coupling can arise in needling treatments from different numbers or arrays of barbed needles, from different barbed needle puncture speeds or frequencies, from different sizes of barbs or needles, from the like, or from some combination thereof. Without being bound by theory, it is believed that the repeated action of the barbed needles may effectively entangle or interweave the fibers of one or the layers with either the fibers or pore structure of the other layer, resulting in effective coupling. Furthermore, more than two layers can be effectively coupled by needling. For example, in a first step, coupling can be attained by co-feeding a top layer and a bottom layer through a needling apparatus to form a multi-layer composition. Then, by controlling the depth of needling, the top layer of the multi-layer composite, which represents the lower "layer" in this second step, can be coupled to a (fibrous) upper layer by co-feeding both to the needling apparatus and by ensuring that the needling depth does not exceed the thickness of the top layer of the composite, thereby forming a three-layer composite formed by two couplings via the needling apparatus. This process can be repeated with any desired number of layers to attain more highly layered composites. Additionally or alternatively, it is possible in multi-layered composites having at least two couplings for at least one of the couplings can be accomplished via needling, while at least one of the other couplings can be accomplished via a different coupling method, such as via lamination.

In some embodiments, the dressing layer 110 can be configured to exhibit substantially elastic recovery under tissue treatment conditions. In some embodiments, the dressing 100 can be configured to exhibit substantial elastic recovery under tissue treatment conditions. In various embodiments, however, at least one of the component layers of the dressing 100 may not exhibit substantially elastic recovery under tissue treatment conditions. For instance, in some embodiments, the dressing layer 110 may include a composite island structure, which may comprise an elastic layer, such as a polyurethane layer, foam layer, silicone layer, or an extensible non-woven layer, coupled to an absorbent layer that is not elastic. Examples of elastic polyurethane layers include polyurethane foam, polyurethane film, melt-blow polyurethane, and thermoplastic polyurethane (such as the Daltex® Stretch from Don & Low Ltd.). In this way, the elastic layer can enable the composite island, and thus the dressing 100, to exhibit substantially elastic recovery at tissue treatment conditions, which may be a desirable characteristic in combination with an ability to absorb generous amounts of liquid, such as saline or exudate. This may be particularly important when the ability to absorb liquid comes mainly from a layer that does not exhibit substantial elastic recovery.

In some embodiments, the dressing layer 110 can comprise a single layer comprising absorbent gelling fibers, for example gelling cellulosic or cellulose ether fibers such as gelling carboxymethyl cellulose fibers. Such absorbent fibers may typically be non-woven and may typically not exhibit substantial elastic recovery under tissue treatment conditions. In such situations, the absorbent fibers of the dressing layer 110 can be coupled to an elastic layer to enable the dressing 100 to exhibit substantially elastic recovery at tissue treatment conditions. If that elastic layer is not part of a composite island in the dressing layer, then in some embodiments it can include or be the backing layer 120, as shown in Figure 1, or the adherent layer or portion thereof disposed between the dressing layer 110 and the backing layer 120. The ability to absorb generous amounts of liquid, such as saline or exudate, and to exhibit substantial elastic recovery under tissue treatment conditions can be a particularly advantageous combination for a dressing 100.

In these contexts, a material that exhibits substantially elastic recovery under tissue treatment conditions is termed "elastic", and a material that does not exhibit substantially elastic recovery under tissue treatment conditions is termed "not elastic". In some embodiments, the dressing 100 or any layer(s) thereof such as the dressing layer 110, having a width and a length perpendicular to the width, may be considered elastic if it exhibits at most about 10%, advantageously about 5% or less or about 2% or less, permanent deformation when subjected to about 50% strain, relative to the length, for about 3 days at that strain level, using an Instron™ mechanical testing machine, for example, at an initial imposed strain rate of about 1% elongation per second up to the total strain value, at which point it can be held for the total strain time. Additionally or alternatively, in some embodiments, the dressing 100 or any layer(s) thereof such as the dressing layer 110, having a width and a length perpendicular to the width, may be considered elastic if it exhibits at most about 5%, advantageously about 2% or less or about 1% or less, permanent deformation when subjected to about 25% strain, relative to the length, for about 24 hours at that strain level, using an Instron™ mechanical testing machine, for example, at an initial imposed strain rate of about 10% elongation per minute up to the total strain value, at which point it can be held for the total strain time. Additionally or alternatively, in some embodiments, the dressing 100 or any layer(s) thereof such as the dressing layer 110, having a width and a length perpendicular to the width, may be considered elastic if it exhibits at most about 1%, and advantageously about 0%, permanent deformation when subjected to about 10% strain, relative to the length, for about 10 minutes at that strain level, using an Instron™ mechanical testing machine, for example, at an initial imposed strain rate of about 1% elongation per minute up to the total strain value, at which point it can be held for the total strain time. Instron™ testing may be performed at room temperature, such as∼20-25°C, and at low relative humidity, for example ∼40% RH or less. Even though particular values are specified with respect to the strain test, materials, layers, or compositions may be considered elastic if optionally tested with one or more parametric deviations, including but not limited to: being conducted at a greater strain than specified (for example, between about 50% strain and about 100% strain, between about 25% strain and about 75% strain, or between about 10% strain and about 50% strain), relative to the length; being conducted for a longer time than specified (for example, between about 10 minutes and about 120 hours, between about 24 hours and about 96 hours, or between about 3 days and about 7 days) at the total strain level; and being conducted at an initial strain rate greater specified (for example, between about 1% elongation per minute and about 600% elongation per minute, between about 10% elongation per minute and about 1200% elongation per minute, or between about 1% elongation per second and about 40% elongation per second).

Substantial elastic recovery may be particularly important for treating tissue in areas of relatively high articulation or flexure, such as proximal to shoulder, elbow, knee, ankle, or hip joints (particularly knee or elbow joints). It may be desirable for the dressing 100 to be able to undergo significant local flexure and substantially retain its shape, and its contact with the tissue site, upon significant articulation. Maintaining shape or tissue contact can reduce the frequency of necessitated dressing changes, reduce tissue blistering from improper contact, increase exudate absorption, reduce healing time, increase patient comfort, or combinations thereof.

An absorbent layer is typically present in the dressing layer 110 either as the sole layer or as one or more of at least two layers. For example, the dressing layer 110 may comprise a composite island having one or more absorbent layers. The absorbent layer may comprise a non-woven material of predominantly non-woven fibers, in some embodiments. For example, the dressing layer 110 may comprise a composite island having one or more absorbent layers. The absorbent layer may comprise a non-woven material of predominantly non-woven fibers such as gelling fibers, in some embodiments. For example, in various embodiments, the absorbent layer may comprise from about 45 parts to about 100 parts by weight of cellulosic (for example, cellulose ether) fibers and optionally up to about 55 parts by weight of reinforcing fibers. In particular embodiments, the absorbent layer may comprise from about 45 parts to about 95 parts by weight, from about 45 parts to about 90 parts by weight, from about 50 parts to about 90 parts by weight, from about 60 parts to about 90 parts by weight, from about 65 parts to about 85 parts by weight, or from about 70 parts to about 90 parts by weight of cellulosic fibers and about 55 parts to about 10 parts by weight, from about 50 parts to about 10 parts by weight, from about 45 parts to about 10 parts by weight, from about 40 parts to about 10 parts by weight, from about 35 parts to about 15 parts by weight, from about 30 parts to about 10 parts by weight, from about 30 parts to about 15 parts by weight, or from about 25 parts to about 10 parts by weight of reinforcing fibers. In some optional embodiments, biodegradable components may additionally be present in the absorbent layer, for example in amounts from about 1 part to about 20 parts by weight, such as from about 1 part to about 15 parts by weight or from about 1 part to about 10 parts by weight. In some embodiments where the dressing layer 110 contains absorbent fibers as the sole layer, the absorbent fibers may be comprised of about 80 parts to about 100 parts by weight of cellulosic, for example cellulose ether such as carboxymethyl cellulose, fibers and optionally up to about 20 parts of reinforcing fibers, biodegradable components, or both.

In some multi-layer embodiments, a surface of the absorbent layer not coupled to the elastic layer can be oriented to be a lower layer, whereas, in other multi-layer embodiments, a surface of the elastic layer not coupled to the absorbent layer can be oriented to be a lower layer. In many embodiments, one or more of the elastic layers may comprise or be a polyurethane layer, a foam layer, or a silicone layer. In some embodiments, it may be advantageous for the absorbent layer to be separated from a tissue site, for example, such that the surface of the elastic layer not coupled to the absorbent layer can be oriented to be a lower layer.

If cellulosic fibers are present in the absorbent layer, the cellulosic fibers may be composed of at least one of carboxymethyl cellulose (CMC), carboxylethyl cellulose, hydroxyethyl cellulose, hydroxypropyl cellulose, hydroxypropylmethyl cellulose, and cellulose ethyl sulphonate (CES) (particularly carboxymethyl cellulose), for example. In some embodiments, the cellulosic component may be at least partially in a salt form, for example, comprising a physiologically acceptable cation such as sodium. CMC is commercially available from a variety of sources, such as under the tradenames Walocel™ (sold by The Dow Chemical Company) and Cekol® (sold by CP Kelco). If reinforcing fibers are present in the absorbent layer, for example, the reinforcing fibers may be composed of at least one of non-gelling cellulose, a polyurethane gel, an amide polymer such as Nylon 6,6, an olefin polymer such as HDPE, an ester polymer such as PET, and a modified acrylamide polymer. If biodegradable components are present in the absorbent layer, for example, the biodegradable components may be composed of, but not limited to, an alginic acid, an alginate salt, chitosan, chitin, a guar gum, a locust bean gum, a xanthan gum, a karaya gum, gelatin, pectin, a starch derivative, a glycosaminoglycan, a galactomannan, a chondroitin salt, heparin, a heparin salt, collagen, oxidized regenerated cellulose (ORC), hyaluronic acid, a hyaluronate salt, or a combination thereof. For such listed salt components, the salt components may include any reasonable counterions, such as sodium, calcium, ammonium, or the like, or combinations thereof. The biodegradable component(s) can be, for example, in the form of a film or a foam, such as an open-cell foam, a reticulated foam, or combinations thereof. If in foam form, the average pore size may vary according to needs of a prescribed therapy, for example, from about 400 microns to about 600 microns). Other physicochemical properties of biodegradable components, such as tensile strength, may be chosen or manipulated, for example, to be suitable to needs of a prescribed treatment.

In some embodiments, particularly if biodegradable components are included, the dressing layer 110 may be characterized as having some biodegradable character or as exhibiting biodegradability. "Biodegradable" and "biodegradability" may individually or collectively refer to a characteristic of a material to disintegrate, degrade, or dissolve upon exposure to physiological fluids or processes, for example, when the dressing layer 110 is positioned with respect to a tissue site. For example, in some embodiments, the dressing layer 110 or a material from which the dressing layer 110 is formed may form a gel when contacted with an aqueous medium, such as water, saline, blood, or exudate. Such biodegradability may be exhibited as a result of chemical process or condition, a physical process or condition, or some combination thereof. For example, the biodegradable characteristics of the dressing layer 110 may substantially reduce or eliminate the need to remove the dressing layer 110 from a tissue site to which it is applied. In some embodiments, at least about 90% by weight of the biodegradable component (particularly at least about 95% by weight, at least about 99% by weight, or about 100% by weight) may be disintegrated, degraded, or dissolved with in a time period of from about 15 days to about 24 hours (particularly from about 12 days to about 36 hours or from about 10 days to about 48 hours), from introduction into a physiological environment when incubated with simulated physiological fluid at a temperature of about 37°C.

In some embodiments, the fibers in the absorbent layer may be deposited on another layer by an electrospinning process. In embodiments where the dressing layer 110 is a single layer comprising absorbent fibers, the other layer can be a backing layer 120 or an adherent layer disposed on the backing layer 120.

In some electrospinning processes, material such as gelling fiber material can typically be in liquid form, for example as dissolved into solution with a solvent or in a melt. For cellulose ether materials, exemplary solvents can include, but are not limited to, C₁-C₆ alcohols such as methanol, ethanol, ethylene glycol, n-propanol, isopropanol, glycerol, n-butanol, sec-butyl alcohol, isobutanol, 1,2-butanediol, 1,4-butanediol, n-pentanol, 2-pentanol, isopentanol, isoamyl alcohol, cyclopentanol, n-hexanol, 2-hexanol, 3-hexanol, cyclohexanol, phenol, or combinations thereof, optionally with other co-solvents.

In large scale electrospinning processes, the liquid or solution may be held in a vessel such as a tank and may be pushed or pulled through an orifice at a given flow rate, while a current or voltage is applied to the orifice, generating a charge build-up in the liquid or solution. The liquid or solution can be driven toward a substrate that sits between the orifice and an oppositely-charged conductive plate to form an electric field. For smaller scale processes, the vessel can be a syringe, and the orifice can be a syringe tip. It is believed that, by application of an appropriately high current or voltage, such as from about 5 kV to about 50 kV, the surface tension of the solution can be overcome by electrostatic repulsion and the material in solution can be passed through the orifice. Once passed through the orifice, the solvent can be evaporated to form fibers of the gelling material as the fibers are accelerated toward a substrate, such as the backing layer 120 or an adherent layer disposed on the backing layer 120.

Because the electrospinning process is designed to convert from liquid or solution to fiber, partial dissolution of such material in the solvent to form a colloid or emulsion may be an acceptable alternative to complete dissolution, so long as the partial dissolution is sufficient to allow flow through the orifice at reasonable currents, voltages, or electric field strengths. In some embodiments, the absorbent fibers can be disposed on a central portion of backing layer 120 or of an adherent layer disposed on the backing layer 120, so as to leave a backing layer margin, for example to enable the dressing 100 to be adhered to a tissue site or near a tissue site.

In some embodiments, the backing layer margin can be protected from deposition by use of a mask oriented between the orifice and the backing layer margin, but leaving the central portion available for fiber deposition. Without being bound by theory, it is believed that the deposition of absorbent fibers by electrospinning onto the backing layer 120 or an adherent layer disposed on the backing layer 120 can form an open fibrous network that, when coupled to the backing layer 120 or the adherent layer, may assist in transferring the quality of substantially elastic recovery to the dressing layer 110 that would otherwise not exhibit substantially elastic recovery.

### Optional Dressing Layer Additives

In some embodiments, the dressing 100, and particularly the dressing layer 110, may optionally comprise one or more additional materials. Such optional components may include, for example, active materials such as preservatives, stabilizing agents, plasticizers, matrix strengthening materials, dyestuffs, and combinations thereof.

Additionally or alternatively, the dressing 100, and particularly the dressing layer 110, may comprise one or more additional active materials, for example, antimicrobial agents that may be effective to aid in wound healing. Non-limiting examples of such active materials may include non-steroidal anti-inflammatory drugs such as acetaminophen, steroids, antimicrobial agents such as penicillins or streptomycins, antiseptics such as chlorhexidine, growth factors such as fibroblast growth factor or platelet derived growth factor, and other well-known therapeutic agents, alone or in combination. If present, such active materials may typically be included at any effective level that show therapeutic efficacy, while preferably not being at such a high level as to significantly counteract any critical or desired physical, chemical, or biological property of the dressing. Depending upon the therapeutic goal(s), the active material(s) may be loaded at a level of from about 10 wppm to about 10 wt% of the layer(s) in which it(they) are present, for example, from about 50 wppm to about 5 wt% or from about 100 wppm to about 1 wt%.

In some embodiments, the antimicrobial agents may comprise a safe and effective amount of poly(hexamethylene biguanide) ("PHMB"), which is also known as polyaminopropyl biguanid ("PAPB") and polyhexanide, having the following general formula. 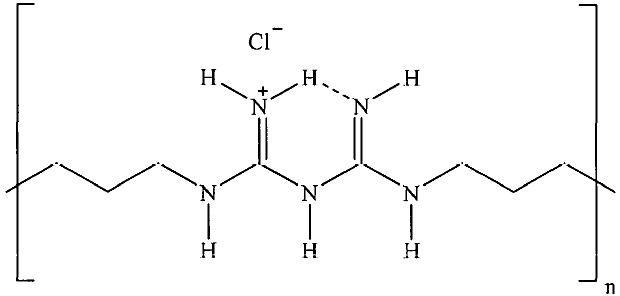 PHMB can be a cationic broad spectrum antimicrobial agent. PHMB may be synthesized by a variety of methods, including polycondensation of sodium dicyanamide and hexamethylenediamine. PHMB is commercially available from a variety of sources. In some embodiments, the PHMB may be present in one or more of the dressing layers at a level of from about 0.005 wt% to about 0.025 wt% of each layer in which it is present, particularly from about 0.007 wt% to about 0.2 wt% or from about 0.008 wt% to about 0.012 wt%, or in some cases at about 0.01 wt%. In some embodiments, the PHMB may be present in one or more of the dressing layers at a level of from about 0.05 wt% to about 3 wt% of each layer in which it is present, particularly from about 0.1 wt% to about 2.5 wt%, from about 0.3 wt% to about 2 wt%, from about 0.5 wt% to about 1.5 wt%, or in some cases at about 1 wt%. In alternative embodiments, silver compounds having antimicrobial efficacy may completely or partially replace the PHMB, as desired. In alternative embodiments, silver compounds having antimicrobial efficacy may completely or partially replace the PHMB, as desired.

In some embodiments where CMC is not already present, the composition may comprise CMC as a modifier for one or more characteristics of the dressing or dressing layer(s), for example, the rheological, absorbency, and other structural characteristics. CMC may be present in the layer(s) at any level appropriate to result in the desired absorbency, rheological, or other structural characteristics of the dressing.

In some embodiments, the dressing layer 110 may contain a strengthening material, which can improve the handling characteristics of the dressing 100, for example, by decreasing its susceptibility to tearing. The strengthening material may comprise non-gelling cellulose fibers in some examples. Such non-gelling cellulose fibers may be substantially water insoluble and may be produced from cellulose that has not been chemically modified to increase water solubility, for example, as contrasted from carboxymethyl cellulose or other cellulose ethers. Non-gelling cellulose fibers are commercially available, such as under the tradename TENCEL (sold by Lenzing AG). In some embodiments, such fibers may be processed from a commercially-available continuous length, by cutting into lengths from about 0.5 to about 5 cm or from about 2 to about 3 cm in length. The non-gelling cellulose fibers may be present in the composition at any level appropriate to result in the desired physical characteristics of the composition. In general, when present, the non-gelling cellulose fibers may comprise from about 1% to about 55% of the layer by weight, particularly from about 5% to about 40% of the layer by weight or from about 10% to about 25% of the layer by weight. In some embodiments, if present, the non-gelling cellulose fibers can be characterized as an additional or alternative reinforcing fiber and can be present in reinforcing fiber amounts.

### Dressing - Cover (Backing Layer)

In most embodiments, aside from dressing layer 110, the dressing 100 may comprise one or more additional layers. In various embodiments, such additional layers may perform any of a variety of functions including, for example, adherence of the dressing 100 to a tissue site or to surrounding tissues, increasing structural rigidity of the dressing 100, imparting substantial elastic recovery through coupling to a layer that would otherwise not exhibit substantial elastic recovery, protection from moisture or other contaminants in the external environment, protection of a tissue surface, delivery of one or more active or other materials to a tissue surface, or combinations thereof. In various embodiments, the additional layers may be conformable to a tissue surface, for example, being capable of conforming such that the appropriate surfaces of the dressing 100 are in substantial contact with a tissue site.

For example, in the embodiment of Figure 1, the dressing 100 comprises a backing layer 120, which may be positioned over the dressing layer 110, for example, so as to cover the dressing layer 110 at a tissue site. The backing layer 120 may have a first surface and a second surface. The backing layer 120 may support the dressing layer 110 on the second surface of the backing layer 120, for example, such that a first surface of the dressing layer 110 can be proximate to the second surface of the backing layer 120. In some embodiments, the first surface of the dressing layer 110 may be in contact with and adhered to the second surface of the backing layer 120.

In particular embodiments, the backing layer 120 of the dressing 100 may extend beyond the boundaries or edges of the dressing layer 110, so as to exhibit an exposed backing layer margin, which may typically be exhibited on the second surface of the backing layer 120. In some embodiments, the backing layer 120 itself can be non-adherent.

In some embodiments, the backing layer 120 may generally be configured to provide a barrier to microbes, a barrier to external contamination, and protection from physical trauma. For example, the backing layer 120 may be constructed from a material that can reduce evaporative losses and provide a fluid seal between two components or two environments, such as between a therapeutic environment and a local external environment. The backing layer 120 may be formed from a suitable material, such as a polymer, for example, which may comprise or be an elastomeric film or membrane that can provide a seal at a tissue site. In some embodiments, the backing layer 120 may comprise or be a polyurethane. In some embodiments, the backing layer 120 may have a high moisture-vapor transmission rate (MVTR). For example, in such an embodiment, the MVTR may be at least 300 g/m² per twenty-four hours. For example, the backing layer 120 may comprise a polymer drape, such as a polyurethane film, that may be permeable to water vapor but generally impermeable to liquid water. In some embodiments, the backing or drape may have a thickness in the range of about from about 15 to about 50 microns.

### Dressing - Secondary layer

Additionally, in some embodiments, the dressing 100 may further comprise a secondary layer, for example, positioned between the dressing layer 110 and the backing layer 120. In some embodiments, the secondary layer may comprise fluid pathways interconnected so as to improve distribution or collection of fluids. For example, in some embodiments, the secondary layer may be a porous material having a plurality of interconnected cells or pores. Suitable examples of porous material include a cellular foam such as an open-cell foam, a reticulated foam, or porous tissue collections. Other suitable porous material may include gauze or felted mat, which generally include pores, edges, or walls adapted to form interconnected fluid pathways. For example, in some embodiments, the secondary layer may be a foam having pore sizes in a range of 400-600 microns. In one non-limiting example, the secondary layer may be reticulated polyurethane foam.

In some embodiments having a secondary layer, the secondary layer may be characterized as exhibiting absorbency. For example, the secondary layer may exhibit an absorbency of at least 3 g saline/g, particularly at least 5 g saline/g, from 5 to 50 g saline/g, from 8 to 40 g saline/g, or from 8 to 20 g saline/g. In some embodiments, the secondary layer may be hydrophilic. In an example in which the secondary layer may be hydrophilic, the secondary layer may also wick fluid away from a dressing layer 110. In such embodiments, the wicking properties of the secondary layer may draw fluid away from dressing layer 110 by capillary flow or other wicking mechanisms. An example of a hydrophilic foam is a polyvinyl alcohol, open-cell foam. Other hydrophilic foams may include those made from or containing a polyether or a polyurethane. Additional or alternative foams that may exhibit hydrophilic characteristics include hydrophobic foams that have been treated or coated to provide hydrophilicity.

### Other Optional Dressing Layers

In some embodiments, the first surface of the dressing layer 110 may be in contact with and adhered to the second surface of the backing layer 120. This adherence may, in some embodiments, result from an adherent layer disposed between the dressing layer 110 and the backing layer 120, thus constituting direct adherence. Such direct adherence means that the adherent layer, or at least the portion disposed between the dressing layer 110 and the backing layer 120, can be comprised of one or more different kinds of physical or chemical adhesive compositions. The adherent layer or portion thereof disposed between the dressing layer 110 and the backing layer 120, in some embodiments, would not be expected to directly contact a tissue site. In embodiments with a backing layer margin extending beyond the dressing layer 110, the adherent layer may typically extend out to cover all or part of the backing layer margin. In such embodiments, the portion of the adherent layer on the margin may adhere the dressing 100 to a tissue site or tissue proximal thereto. Adherents that may directly contact tissue or that may be exposed to a treatment environment can typically have additional requirements, such as biocompatibility, and may be selected from a smaller list of physical or chemical adhesive compositions.

In some embodiments, such as where an adherent layer is an external layer in the dressing 100, for example to adhere the dressing 100 to a tissue site, the adherent layer can be releasably coupled to a release liner configured for removal before application to a tissue site, for example.

Adherence between the dressing layer 110 and the backing layer 120 may additionally or alternatively be indirect. For example, in some embodiments with a backing layer margin extending beyond the dressing layer 110, the adherent layer may be disposed on the backing layer margin and extend further over some portion of the dressing layer 110, such as the margin of the dressing layer 110. If this occurs without an adherent layer between the backing layer 120 and the dressing layer 110, the adherent layer may be said to indirectly adhere those layers, because those layers are each adhered to the adherent layer but not directly to each other. Such a configuration can allow an absorbent portion of the dressing layer 110 to expand differentially from the backing layer 120, for instance enabling relatively high levels of absorption of fluid with additional degrees of freedom. If the backing layer 120 is directly adhered to the dressing layer 110, the absorbent portion of the dressing layer 110 may expand into the tissue site, which can in some embodiments create undesirable pressure on a tissue site.

In particular embodiments, the adherent layer can comprise a hydrocolloid material. In particular embodiments, if present, the second adherent layer can comprise a hydrocolloid, a silicone adhesive, or an acrylic adhesive.

In some embodiments, absorbent material may be absent in or removed from a zone within the absorption layer. Such embodiments offer an additional or alternative mechanism enabling at least partial fluid absorptive expansion within the absorption layer, which can enable additional degrees of freedom for fluid absorption while creating no or little additional pressure on the tissue site. For example, by creating a central zone in the absorbent layer of dressing layer 110 that is absent of material, the other portions of the absorbent layer can have extra volume to expand and can optionally experience increased fluid flow within the dressing layer 110, thus rendering the absorbent layer more efficient.

In some embodiments, the absorbent layer may be perforated to increase fluid flow, to reduce time to equilibrium absorption, or both. Such embodiments offer another additional or alternative mechanism enabling additional degrees of freedom for fluid absorption while creating no or little additional pressure on the tissue site.

In some embodiments, the dressing 100 may include a contact layer, which may be non-adherent. For example, a contact layer may be disposed over the second surface of the dressing layer 110, opposite the backing layer 120. Non-adherent contact layers may be particularly advantageous in fibrinous situations to reduce potential adherence of the dressing layer 110 to a tissue site, to inhibit, reduce, minimize, or prevent fibers from the dressing layer 110 or the absorbent layer from shedding into a tissue site, to enable fluid to be effectively drawn away from the tissue site through the contact layer, or any combination thereof. In some embodiments, therefore, the contact layer may be perforated, for example, for increased fluid flow. In various embodiments, the contact layer may comprise at least one of: an alkyl acrylate polymer, such as a methyl acrylate polymer, an ethyl acrylate polymer, or the like; an alkacrylate polymer, such as a methacrylate polymer, an ethacrylate polymer, or the like; and an alkyl alkacrylate polymer, such as a methyl methacrylate polymer, an ethyl methacrylate polymer, a methyl ethacrylate polymer, an ethyl ethacrylate polymer, or the like. Such (alk)acrylate polymers may be homopolymers but are more often copolymers, for example, with olefin comonomers. In particular, the non-adherent layer may comprise an ethylene-methyl acrylate copolymer, such as used in TIELLE™ Dressings and in SILVERCEL™ NON-ADHERENT Dressings available from Systagenix Wound Management, Limited. In various embodiments, the contact layer may comprise a silicone or polysiloxane polymer or copolymer. In such embodiments, the contact layer can extend over the absorbent layer, over the composite island, or over the dressing layer, as applicable, and over at least a portion of the backing layer margin. By extending over at least a portion of the backing layer margin, the portion of the contact layer extending over the backing layer margin may be coupled to the backing layer via the adherent layer. Optionally, the contact layer may have margin perforations, for example through which access can be allowed for the adherent layer to contact a tissue site. Additionally or alternatively, particularly if the contact layer does not have sufficient tack to adhere the dressing to a tissue site, a second adherent layer may optionally be disposed on a surface of the contact layer opposite the backing layer, absorbent layer, composite island, or dressing layer.

### Negative-Pressure Therapy

Additionally, in some embodiments, a dressing layer such as the dressing layer 110, or a dressing comprising such a dressing layer, such as the dressing 100, may be employed in therapy, for example to treat a tissue site with reduced pressure. Treatment with reduced pressure may be commonly referred to as "negative-pressure therapy," but is also known by other names, including "negative-pressure wound therapy," "reduced-pressure therapy," "vacuum therapy," "vacuum-assisted closure," and "topical negative-pressure," for example. Negative-pressure therapy may provide a number of benefits, including migration of epithelial and subcutaneous tissues, improved blood flow, micro-deformation of tissue at a tissue site, and combinations thereof. Individually or together, these benefits may increase development of granulation tissue and reduce healing times.

In various embodiments, a negative-pressure therapy system may generally include a negative-pressure supply, and may include or be configured to be coupled to a distribution component. In general, a distribution component may refer to any complementary or ancillary component configured to be fluidly coupled to a negative-pressure supply in a fluid path between a negative-pressure supply and a tissue site.

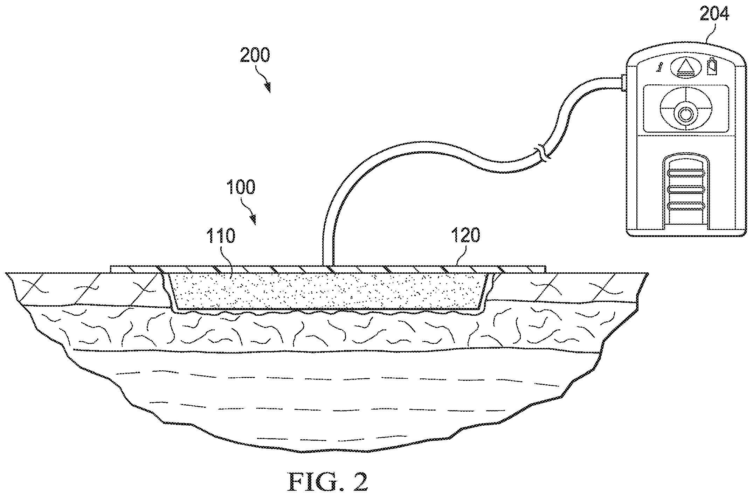

Figure 2 is a simplified schematic of an example embodiment of a therapy system 200 that can provide negative-pressure therapy to a tissue site. In the example embodiment of Figure 2, the dressing 100 is fluidly coupled to a negative-pressure source 204, such that negative pressure may be applied to a tissue site via the dressing 100.

For example, the dressing layer 110 may be generally configured to distribute negative pressure. For example, in some embodiments, the dressing layer 110 may comprise or be configured as a manifold. A "manifold" in this context generally includes any composition or structure providing a plurality of pathways configured to collect or distribute fluid across a tissue site under pressure. For example, a manifold may be configured to receive negative pressure from a negative-pressure source and to distribute negative pressure through multiple apertures or pores, which may have the effect of collecting fluid and drawing the fluid toward the negative-pressure source. More particularly, in the embodiment of Figure 2, the dressing layer 110 is configured to receive negative pressure from the negative-pressure source 204 and to distribute negative pressure across the dressing layer 110. For example, this may have the effect of collecting fluid from a sealed space, such as by drawing fluid from the tissue site through the dressing layer 110. In additional or alternative embodiments, the fluid path(s) may be reversed or a secondary fluid path may be provided to facilitate movement of fluid across a tissue site. In some embodiments, the fluid pathways of a manifold may be interconnected to improve distribution or collection of fluids. In some embodiments, a manifold may comprise or be a porous foam material having a plurality of interconnected cells or pores. For example, open-cell foams generally include pores, edges, walls, or combinations thereof that may form interconnected fluid pathways, such as channels.

The fluid mechanics associated with using a negative-pressure source to reduce pressure in another component or location, such as within a sealed therapeutic environment, may be mathematically complex. However, the basic principles of fluid mechanics applicable to negative-pressure therapy are generally well-known to those skilled in the art. The process of reducing pressure may be described generally and illustratively herein as "delivering," "distributing," or "generating" negative pressure, for example.

In general, a fluid such as exudate flows toward lower pressure along a fluid path. Thus, the term "downstream" typically implies something in a fluid path relatively closer to a source of negative pressure or further away from a source of positive pressure. Conversely, the term "upstream" implies something relatively further away from a source of negative pressure or closer to a source of positive pressure. This orientation is generally presumed for purposes of describing various features and components herein. However, the fluid path may also be reversed in some applications, such as by substituting a positive-pressure source for a negative-pressure source, and this descriptive convention should not be construed as a limiting convention.

As used herein, "negative pressure" is generally intended to refer to a pressure less than a local ambient pressure, such as the ambient pressure in a local environment external to a sealed therapeutic environment provided by the dressing 100. In many cases, the local ambient pressure may also be the atmospheric pressure proximate to or about a tissue site. Additionally or alternatively, the pressure may be less than a hydrostatic pressure associated with the tissue at the tissue site. Unless otherwise indicated, values of pressure stated herein are gauge pressures. Similarly, references to increases in negative pressure typically refer to a decrease in absolute pressure, for example a more negative pressure, while decreases in negative pressure typically refer to an increase in absolute pressure, for example a less negative pressure or a more positive pressure. While the amount and nature of negative pressure applied to a tissue site may vary according to therapeutic requirements, the pressure is generally a low vacuum, also commonly referred to as a rough vacuum, for example between -5 mm Hg (-667 Pa) and -500 mm Hg (-66.7 kPa). Common therapeutic ranges can be between -75 mm Hg (-9.9 kPa) and -300 mm Hg (-39.9 kPa).

In various embodiments, a negative-pressure supply, such as the negative-pressure source 204, may be a reservoir of air at a negative pressure. Alternatively, negative-pressure source 204 may be a manual or electrically-powered device that can reduce the pressure in a sealed volume, such as a vacuum pump, a suction pump, a wall suction port available at many healthcare facilities, or a micro-pump, for example. A negative-pressure supply may be housed within or used in conjunction with other components that further facilitate therapy, such as sensors, processing units, alarm indicators, memory, databases, software, display devices, or user interfaces. For example, in some embodiments, the negative-pressure source 204 may be combined with a controller and other components into a therapy unit. A negative-pressure supply may have one or more supply ports configured to facilitate coupling and de-coupling of the negative-pressure supply to one or more distribution components.

In various embodiments, components may be fluidly coupled to each other to provide a path for transferring fluids between the components. For example, components may be fluidly coupled through a fluid conductor, such as a tube. The term "fluid conductor" is intended to broadly include a tube, pipe, hose, conduit, or other structure with one or more lumina adapted to convey a fluid between two ends thereof. Typically, a fluid conductor may be an elongated, cylindrical structure with some flexibility, but the geometry and rigidity may vary. In some embodiments, the negative-pressure source 204 may be operatively coupled to the dressing 100 via a dressing interface. For example, in the embodiment of Figure 2, the dressing 100 may be coupled to the negative-pressure source 204 via a dressing interface to receive negative pressure.

### Methods of Use

Also disclosed herein are methods of treating a tissue site, for example, in the context of various therapies, such as eliminating, minimizing, or reducing edema, particularly in areas of relatively high articulation or flexure, such as during post-operative care. Non-limiting examples of areas implicating relatively high articulation or flexure include shoulder, elbow, knee, ankle, or hip joints, particularly knee or elbow joints.

In some embodiments, a therapy method may comprise positioning the dressing layer 110 with respect to a tissue site. For example, in operation, the dressing layer 110 may be positioned proximate to the tissue site. The dressing layer 110 may be used with any of a variety of wounds, such as those occurring from trauma, surgery, or disease. For example, the dressing layer 110 may be placed within, over, on, or otherwise proximate to the tissue site. Additionally, in some embodiments, a cover such as the backing layer 120 may be placed over the dressing layer 110 and sealed to an attachment surface near the tissue site. For example, the backing layer 120 may be sealed to undamaged epidermis peripheral to a tissue site. In some embodiments, the dressing layer 110 may be positioned and the backing layer 120 may be positioned after the dressing layer 110 has been positioned. In some embodiments, the dressing layer 110 and backing layer 120 may be preassembled, for example, such that the dressing layer 110 and backing layer 120 are positioned with respect to each other prior to placement proximate the tissue site. Thus, the backing layer 120 can provide a sealed therapeutic environment including the dressing layer 110 and proximate to a tissue site, substantially isolated from the external environment.

Additionally, in some negative-pressure therapy embodiments, a negative-pressure therapy may comprise positioning the dressing layer 110 and backing layer 120 proximate to a tissue site. For example, the various components of the dressing layer 110 may be positioned with respect to the tissue site sequentially or, alternatively, may be positioned with respect to each other and then positioned with respect to the tissue site. The negative-pressure therapy may further comprise sealing the dressing layer 110 to tissue surrounding the tissue site to form a sealed space. For example, the backing layer 120 may be placed over the dressing layer 110 and sealed to an attachment surface near the tissue site, such as undamaged epidermis peripheral to a tissue site. Thus, the dressing layer 110 and backing layer 120 can provide a sealed therapeutic environment proximate to the tissue site, substantially isolated from the external environment.

The negative-pressure therapy method may further comprise fluidly coupling a negative-pressure source to the sealed space and operating the negative-pressure source to generate a negative pressure in the sealed space. For example, the negative-pressure source 204 may be coupled to the dressing 100 such that the negative-pressure source 204 may be used to rcducc the pressure in the sealed space. For example, negative pressure applied across the tissue site via the dressing 100 may be effective to induce macrostrain and microstrain at the tissue site, as well as to remove exudates and other fluids from the tissue site.

### EXAMPLES

One, some, or all of the advantages associated with the disclosed compositions, dressings, methods of making, and methods of using or treating may be further demonstrated by the following, non-limiting examples.

### Example I

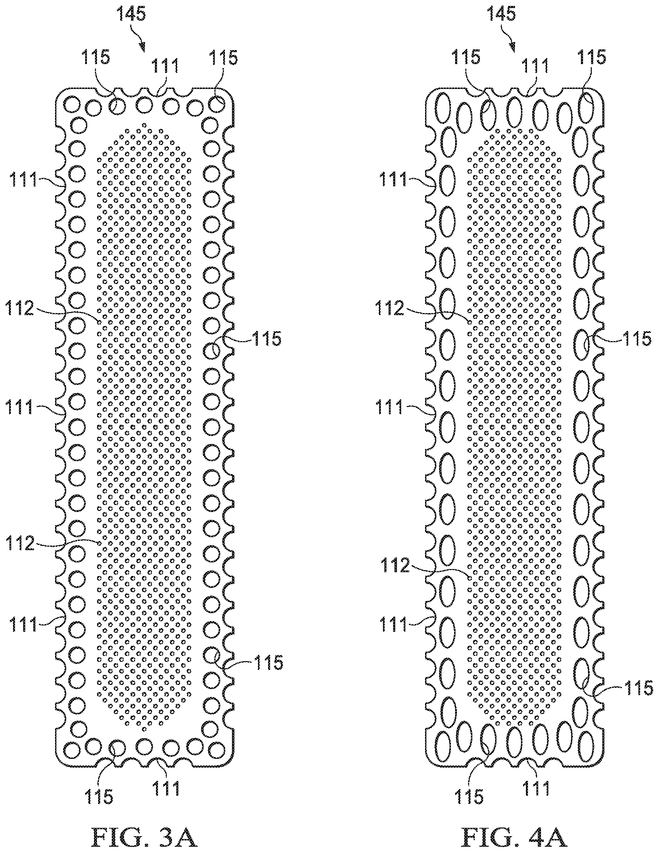

Figure 3A shows an example of a silicone layer 145 that may be associated with some embodiments of the dressing layer 110. As illustrated in Figure 3A, some embodiments of the silicone layer 145 may be perforated. The silicone layer 145 of Figure 3A includes central perforations 112 having a roughly circular shape. The silicone layer 145 may also have margin perforations 115, which may also exhibit a roughly circular shape. In some embodiments, the margin perforations 115 may be considerably larger than the central perforations 112. The silicone layer 145 of Figure 3A also has edge cuts 111, each of the edge cuts 111 having a semi-circular shape.

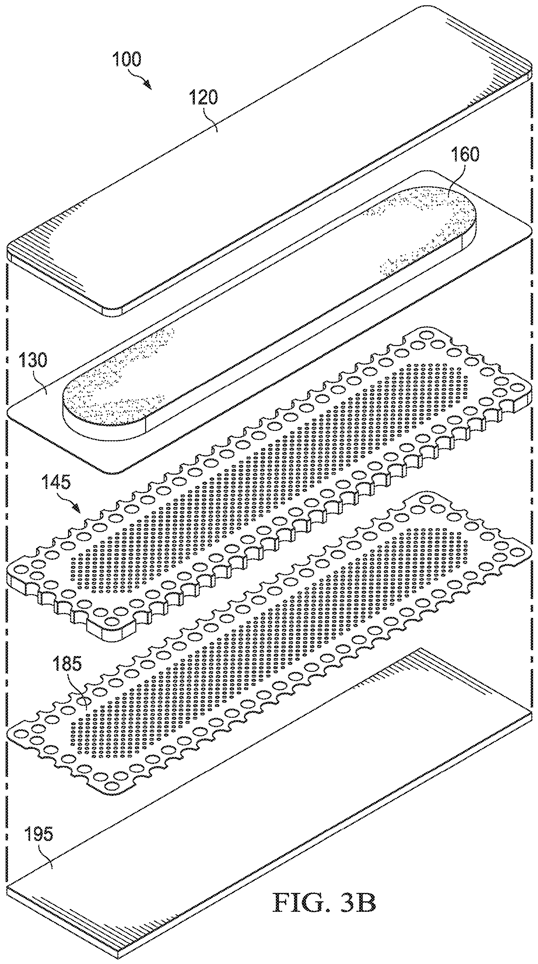

Figure 3B is an exploded view illustrating additional details that may be associated with some examples of the dressing 100 with the silicone layer 145 of Figure 3A. Figure 3B also illustrates an embodiment of the backing layer 120, below which an example of an absorbent layer 160 is shown to be disposed, defining a backing layer margin 125. An example of an adherent layer 130 is shown disposed on the backing layer margin 125 surrounding the absorbent layer 160. The silicone layer 145 is shown to be disposed below the absorbent layer 160 and adhered to adherent layer 130 over substantially the entire backing layer margin 125. Though not explicitly labelled in Figure 3B, the combination of absorbent layer 160 and perforated silicone layer 145, as adhered via adherent layer 130, collectively describes a multi-layer composite structure constituting the dressing layer 110. Backing layer 120 can be composed of a polyurethane in some examples, and the absorbent layer 160 can be a fibrous layer containing a combination of ∼80 wt% CMC fibers and ∼20% reinforcing fibers in some examples. Figure 3B also shows an optional second adherent layer 185 disposed below the silicone layer 145 but not covering the central perforations 112, the margin perforations 115, and the edge cuts 111. In some embodiments, the adherent layer 130, the optional second adherent layer 185, or both can comprise or be an adhesive with a bond strength that reduces when exposed to light, for example DermaTac® commercially available from Surgical Specialties Corporation of Reading, PA. An optional release liner 195 is shown as being disposed below the optional second adherent layer 185 and below the silicone layer 145. The release liner 195 may be included in packaging and removed before applying the dressing 100 to a tissue site.

### Example 2

Figure 4A shows another example of the silicone layer 145 that may be associated with some embodiments of the dressing layer 110. As illustrated in Figure 4A, some embodiments of the silicone layer 145 may be perforated. The silicone layer 145 of Figure 4A includes central perforations 112 having a roughly circular shape. The silicone layer 145 may also have margin perforations 115, which may exhibit a roughly oval shape. In some embodiments, the margin perforations 115 may be considerably larger than the central perforations 112. The silicone layer 145 of Figure 4A also has edge cuts 111, each of the edge cuts 111 having a semi-circular shape.

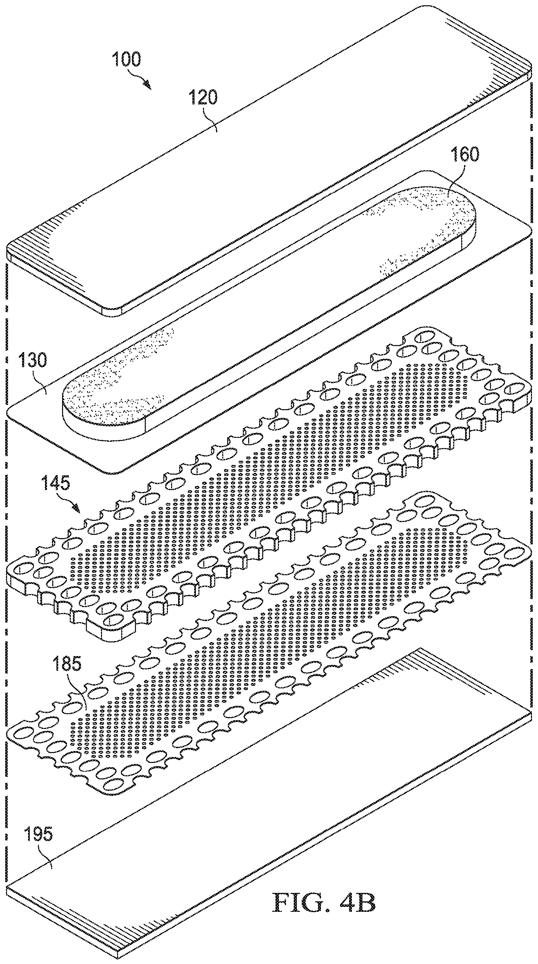

Figure 4B is an exploded view illustrating additional details that may be associated with an example embodiment of the dressing 100 with the perforated silicone layer 145 of Figure 4A. Figure 4B also illustrates an example of the backing layer 120, below which an example of the absorbent layer 160 is shown to be disposed, defining a backing layer margin 125. An example of an adherent layer 130 is shown disposed on the backing layer margin 125 surrounding the absorbent layer 160. The silicone layer 145 is shown to be disposed below absorbent layer 160 and adhered to adherent layer 130 over substantially the entire backing layer margin 125. Though not explicitly labelled in Figure 4B, the combination of absorbent layer 160 and perforated silicone layer 145, as adhered via adherent layer 130, collectively describes a multi-layer composite structure constituting the dressing layer 110. Backing layer 120 can be composed of a polyurethane in some examples, and absorbent layer 160 can be a fibrous layer containing a combination of ∼80 wt% CMC fibers and ∼20% reinforcing fibers in some examples. Figure 4B also shows an optional second adherent layer 185 disposed below the silicone layer 145 but not covering the central perforations 112, the margin perforations 115, and the edge cuts 111. In some embodiments, the adherent layer 130, the optional second adherent layer 185, or both can comprise or be an adhesive with a bond strength that reduces when exposed to light, for example DermaTac® commercially available from Surgical Specialties Corporation of Reading, PA. An optional release liner 195 is shown as being disposed below the optional second adherent layer 185 and below the silicone layer 145. The release liner 195 may be included in packaging and removed before applying the dressing 100 to a tissue site.

### Example 3

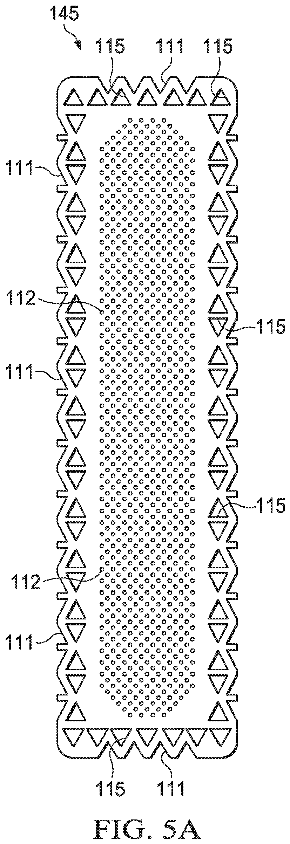

Figure 5A shows another example of the silicone layer 145 that may be associated with some embodiments of the dressing layer 110. As illustrated in Figure 5A, some embodiments of the silicone layer 145 may be perforated. The silicone layer 145 includes central perforations 112 having a roughly circular shape. The silicone layer 145 may also have margin perforations 115 having a roughly triangular shape, alternating in orientation such that triangular sides and triangular point are alternately proximal to each other. In some embodiments, the margin perforations 115 may be considerably larger than the central perforations 112. The silicone layer 145 of Figure 5A also has edge cuts 111, each having a shape that is roughly half the margin perforations 115 and reflecting the same orientation or pattern.

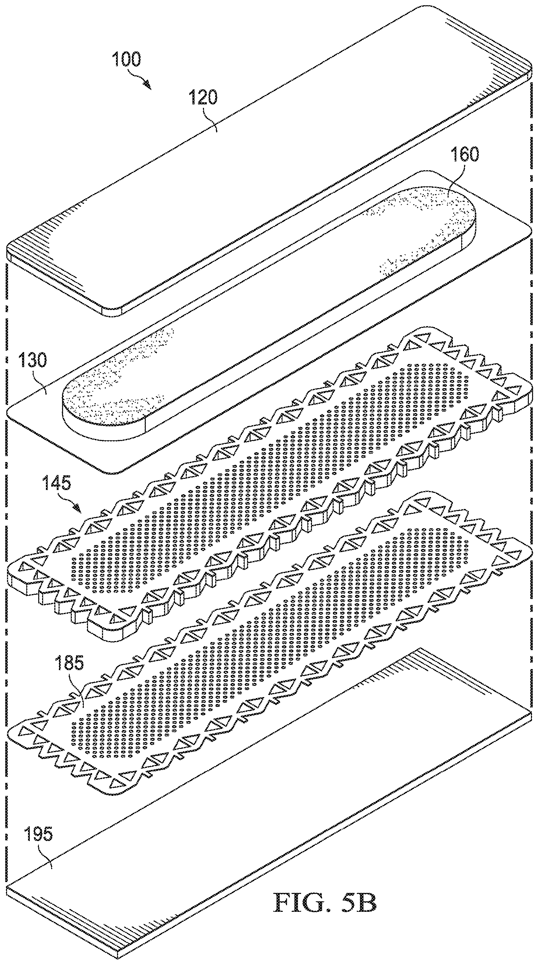

Figure 5B is an exploded view showing additional details that may be associated with some examples of the dressing 100 with the silicone layer 145 of Figure 5A. Figure 5B also illustrates an embodiment of the backing layer 120, below which an example of an absorbent layer 160 is shown to be disposed, defining a backing layer margin 125. An example of an adherent layer 130 is shown disposed on the backing layer margin 125 surrounding the absorbent layer 160. The silicone layer 145 is shown to be disposed below absorbent layer 160 and adhered to adherent layer 130 over substantially the entire backing layer margin 125. Though not explicitly labelled in Figure 5B, the combination of absorbent layer 160 and perforated silicone layer 145, as adhered via adherent layer 130, collectively describes a multi-layer composite structure constituting the dressing layer 110. Backing layer 120 can be composed of a polyurethane in some examples, and absorbent layer 160 can be a fibrous layer containing a combination of ∼80 wt% CMC fibers and ∼20% reinforcing fibers in some examples. Figure 5B also shows an optional second adherent layer 185 disposed below the silicone layer 145 but not covering the central perforations 112, the margin perforations 115, and the edge cuts 111. In some embodiments, the adherent layer 130, the optional second adherent layer 185, or both can comprise or be an adhesive with a bond strength that reduces when exposed to light, for example DermaTac® commercially available from Surgical Specialties Corporation of Reading, PA. An optional release liner 195 is shown as being disposed below the optional second adherent layer 185 and below the silicone layer 145. The release liner 195 may be included in packaging and removed before applying the dressing 100 to a tissue site.

### Example 4

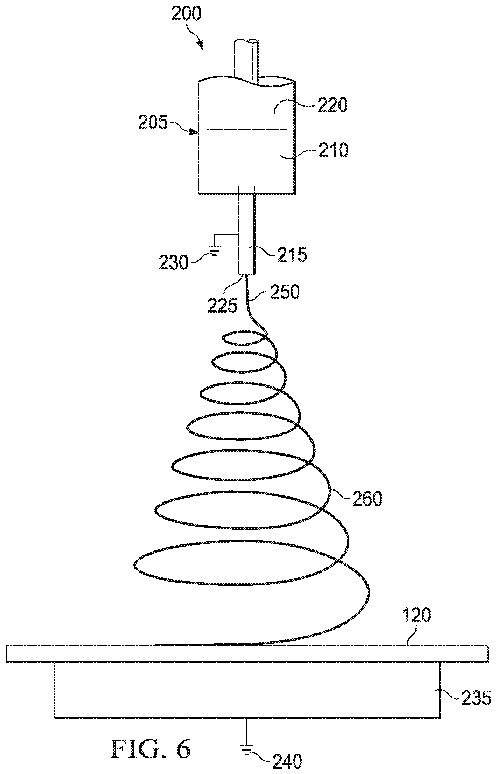

Figure 6 shows a diagram of an electrospinning system 200. The electrospinning system 200 is for a smaller-scale electrospinning process comprising a syringe 205 containing carboxymethyl cellulose material dissolved in a C₁-C₆ alcohol, such as ethanol or isopropanol (collectively CMC solution 210). The syringe 205 also comprises a syringe tip 215 and an orifice 225, through which the CMC solution 210 may flow. Flow may be aided by a syringe piston (not shown) exerting pressure 220, for example relatively constant pressure, on the CMC solution 210. A current or voltage 230 is applied to the syringe tip 215 in order to apply a charge to the CMC solution. An electric field can be generated by application of a complementary current or voltage 240 to a conductive plate 235 located below a deposition substrate, in this case backing layer 120, or adherent layer (not shown) coupled to a surface of backing layer 120 opposite the conductive plate 235. In an electrospinning process, the CMC solution 210 may flow through the orifice 225 of the syringe tip 215 by the exerted pressure 220, by the electric field created through application of current or voltage 230 to the syringe tip 215 and of complementary current or voltage 240 to conductive plate 235, or by both. Initially, a liquid stream 250 exits the orifice 225 containing both the solvent and the carboxymethyl cellulose material of the CMC solution, being drawn toward the backing material 120 or the adherent layer (not shown) disposed thereon. Once passed through the orifice 225, the solvent in the liquid stream 250 can be at least partially evaporated to form CMC gelling fibers, at which point the stream becomes a solid stream 260 containing CMC gelling fibers. In Figure 6, the solid stream 260 is shown as a continuous fiber, which is believed to swirl due to a whipping process caused by a surface electrostatic repulsion due to the electric field created by 230 and 240. The solid stream 260 of CMC gelling fibers are then deposited on the backing layer 120 or on an adherent layer disposed on the backing layer 120, preferably on a central portion thereof, thereby leaving a backing layer margin.

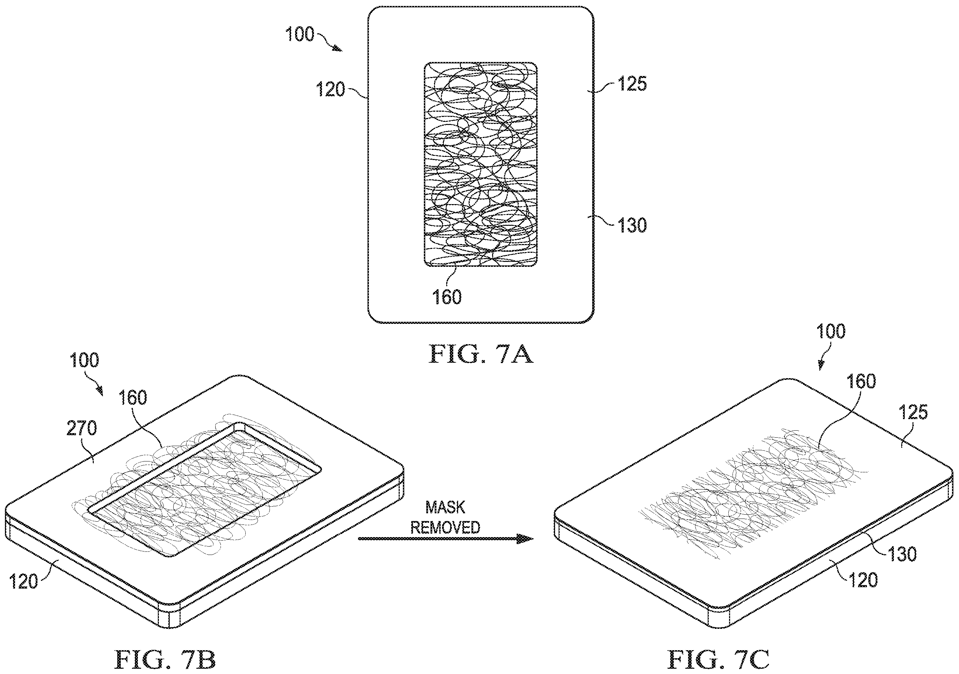

Figure 7B shows the use of a mask 270 over the backing layer 120 or on an adherent layer (not shown) disposed on the backing layer 120 in an electrospinning process using the electrospinning system 200 of Figure 6. The use of mask 270 can limit the deposition of CMC fibers as absorbent layer 160 onto the central substrate region of the backing layer 120 or of the adherent layer (not shown) disposed between absorbent layer 160 and the backing layer 120. Whether there is already an adherent layer disposed thereon, an adherent layer will typically be disposed on and coupled to the backing layer 120 on at least the backing layer margin 125, for example to enable the dressing 100 to be adhered to a tissue site or near a tissue site. Figure 7A shows dressing 100 with an adherent layer disposed only on this backing layer margin 125 (backing layer 120 behind this perspective view and thus not shown), with the absorbent fibers deposited in absorbent layer 160 on the central portion thereof. Alternatively, Figure 7C shows the adherent layer 130 disposed over the entire backing layer 120, with the absorbent fibers of absorbent layer 160 deposited over the central portion of the adherent layer 130, thereby leaving the backing layer margin 125 covered by adherent layer 130. In Figure 7C, the adherent layer 130 of dressing 100 is necessarily disposed over the backing layer margin 125 before the absorbent fibers are deposited thereon, whereas the adherent layer 130 of dressing 100 in Figure 7A can be disposed over the backing layer margin 125 either before application of the mask 270 or after, as desired.

### Non-limiting discussion of terminology

The description and specific examples above are provided for illustration only and are not intended to limit the scope of the claimed subject matter. Moreover, recitation of multiple embodiments having stated features does not exclude other embodiments having additional features, or other embodiments incorporating different combinations of the stated features. Components may also be combined or eliminated in various configurations for purposes of sale, manufacture, assembly, or use. Specific examples are provided for illustrating how to make and use the compositions, and examples of methods are not intended to be a representation that given embodiments have, or have not, been made or tested. Equivalent changes, modifications and variations of some embodiments, materials, compositions and methods can be made within the scope of the appended claims, with substantially similar results.

As used herein, the words "include," "contain," and their variants, are intended to be non-limiting, such that recitation of items in a list is not necessarily to the exclusion of other like items that may also be useful in the materials, compositions, devices, and methods of this technology. Similarly, the terms "can" and "may" and their variants are intended to be non-limiting, such that recitation that an embodiment can or may comprise certain elements or features does not exclude other embodiments that do not contain those elements or features. Moreover, descriptions of various alternatives using terms such as "or" do not require mutual exclusivity unless clearly required by the context, and the indefinite articles "a" or "an" do not limit the subject to a single instance unless clearly required by the context.

Although the open-ended term "comprising," as a synonym of non-restrictive terms such as including, containing, or having, is used herein to describe example embodiments, embodiments may alternatively be described using more limiting terms such as "consisting of" or "consisting essentially of." Thus, for any given embodiment reciting materials, components or process steps with such open-ended terms, similar or analogous embodiments are contemplated consisting of, or consisting essentially of, such materials, components or processes excluding additional materials, components or processes (for consisting of) and excluding additional materials, components or processes affecting the significant properties of the embodiment (for consisting essentially of), even though such additional materials, components or processes are not explicitly recited in this application. For example, recitation of a composition or process reciting elements A, B and C specifically envisions embodiments consisting of, and consisting essentially of, A, B and C, excluding an element D that may be recited in the art, even though element D is not explicitly described as being excluded herein.

Disclosure of values and ranges of values for specific parameters, such as temperatures, molecular weights, weight percentages, etc., are not exclusive of other values and ranges of values useful herein. It is envisioned that two or more specific exemplified values for a given parameter may define endpoints for a range of values that may be claimed for the parameter. For example, if Parameter X is exemplified herein to have value A and also exemplified to have value Z, it is envisioned that parameter X may have a range of values from about A to about Z. Similarly, disclosure of two or more ranges of values for a parameter, whether such ranges are nested, overlapping or distinct, may subsume all possible combination of ranges for the value that might be claimed using endpoints of the disclosed ranges. For example, if parameter X is exemplified herein to have values in the range of 1-10, or 2-9, or 3-8, Parameter X may be envisioned as having other ranges of values including 1-2, 1-3, 1-8, 1-9, 2-3, 2-8, 2-10, 3-9, 3-10, 8-9, 8-10, and 9-10.

The term "about," as used herein, is intended to refer to deviations in a numerical quantity that may result from various circumstances, for example, through measuring or handling procedures in the real world; through inadvertent error in such procedures; through differences in the manufacture, source, or purity of compositions or reagents; from computational or rounding procedures; and other deviations as will be apparent by those of skill in the art from the context of this disclosure. For example, unless otherwise defined by the specification per se or by the context of the specification, the term "about," with reference to a value, may refer to any number that would round to that value, based on a significant digit analysis. In such a circumstance, a value of "about 30%", assuming the "3" is the only significant digit, could encompass from 25% to just below 35%. However, the context of the specification would limit that interpretation based on significant digits, so that the "about" ranges do not overlap. For example, if the specification discloses ranges that include "about 25%, about 30%, about 35%," etc., about 30% *in that context* could encompass from 27.5% to just below 32.5%. Alternatively, the term "about" may refer to deviations that are greater or lesser than a stated value or range by ±10% of the stated value(s), as appropriate from the context of the disclosure. In such a circumstance, a value of "about 30%" may encompass from 27% to 33%.

## Claims

1. A dressing (100) for treating a tissue site, the dressing comprising:
a composite island comprising a polyurethane layer coupled to an absorbent layer (160) comprising non-woven gelling fibers, wherein a surface of the absorbent layer (160) not coupled to the polyurethane layer is oriented to be a lower layer; and
a backing layer (120) extending beyond the absorbent layer (160) to define a backing layer margin (125) and an adherent layer (130) disposed on at least the backing layer margin (125),
wherein:
the polyurethane layer is substantially elastically deformable under tissue treatment conditions,
the absorbent layer (160) is not elastically deformable under tissue treatment conditions, and
the composite island is substantially elastically deformable under tissue treatment conditions.

2. The dressing (100) of claim 1, wherein a means for coupling the polyurethane layer and the absorbent layer (160) is obtainable by co-feeding the polyurethane layer and the absorbent layer (160) through a needle loom machine.

3. The dressing (100) of claim 1 or claim 2, wherein the absorbent layer (160) comprises:
(i) from about 45% to about 90% of cellulose ether gelling fibers; and
(ii) from about 10% to about 55% of reinforcing fibers.

4. The dressing (100) of claim 1 or claim 2, wherein the absorbent layer (160) comprises cellulose ether gelling fibers, and further wherein the polyurethane layer is coupled to the absorbent layer (160) by reinforcing fibers needled through both the absorbent layer (160) and the polyurethane layer.

5. The dressing (100) of claim 3 or claim 4, wherein the cellulose ether gelling fibers are composed of at least one of carboxymethyl cellulose, carboxylethyl cellulose, hydroxyethyl cellulose, hydroxypropyl cellulose, and hydroxypropylmethyl cellulose.

6. The dressing (100) of any of claims 3-5, wherein the reinforcing fibers are composed of non-gelling cellulose, a polyurethane gel, an amide polymer, an olefin polymer, an ester polymer, a modified acrylamide polymer, or a combination or copolymer thereof.

7. The dressing (100) of any of claims 3-6, wherein the absorbent layer (160) further comprises from about 10% to about 40% of an alginate, chitosan, chitin, a guar gum, pectin, a starch derivative, a cellulose derivative, a glycosaminoglycan, a galactomannan, a chondroitin salt, heparin, collagen, oxidized regenerated cellulose (ORC), a heparin salt, hyaluronic acid, a hyaluronic acid salt, or a combination thereof.

8. The dressing (100) of claim 1, wherein the adherent layer (130) further extends over at least a margin of the composite island, thereby fastening the adherent layer (130) to the margin of the composite island.

9. The dressing (100) of any of claims 1-8, further comprising a non-adherent layer disposed on the composite island but not on the adherent layer (130), optionally wherein the non-adherent layer is perforated and comprises an ethylene-methyl acrylate copolymer.

10. The dressing (100) of any of claims 1-9, wherein the backing layer (120) is moisture vapor-permeable, liquid-impermeable, and comprises a polyurethane.

11. The dressing (100) of any of claims 1-10, wherein the adherent layer (130) comprises a hydrocolloid or an acrylic adhesive.

12. The dressing (100) of any of claims 1-11, wherein the polyurethane layer is a melt-blown polyurethane sheet, a thermoplastic polyurethane sheet, or a polyurethane foam.

13. The dressing (100) of any of claims 1-8 and 10-12, further comprising a perforated silicone contact layer (145) extending over at least the backing layer margin (125), optionally extending over the backing layer margin (125) and the composite island.

14. The dressing (100) of claim 13, wherein the perforated silicone contact layer (145) is coupled to the adherent layer (130).

15. The dressing (100) of any of claims 13-14, wherein the perforated silicone contact layer (145) comprises perforations (112, 115) of at least two sizes, optionally wherein silicone contact layer perforations (112, 115) over the composite island are smaller than silicone contact layer perforations (112, 115) over the backing layer margin (125).

16. The dressing (100) of any of claims 13-15, wherein silicone contact layer perforations (112, 115) are elongated in a direction to allow for greater extension of the dressing in said direction.

## Patentansprüche

1. Verband (100) zur Behandlung einer Gewebestelle, wobei der Verband umfasst:
eine Verbundstoff-Insel umfassend eine Polyurethanschicht, die mit einer absorbierenden Schicht (160) gekoppelt ist, die nicht gewebte Gelierfasern umfasst, wobei eine Oberfläche der absorbierenden Schicht (160), die nicht mit der Polyurethanschicht gekoppelt ist, so ausgerichtet ist, dass sie eine untere Schicht ist; und
eine Trägerschicht (120), die sich über die absorbierende Schicht (160) hinaus erstreckt, um einen Trägerschichtrand (125) zu definieren, und eine haftende Schicht (130), die zumindest auf dem Trägerschichtrand (125) angeordnet ist,
wobei:
die Polyurethanschicht unter den Bedingungen der Gewebebehandlung im Wesentlichen elastisch verformbar ist,
die absorbierende Schicht (160) unter den Bedingungen der Gewebebehandlung nicht elastisch verformbar ist und
die Verbundstoff-Insel unter den Bedingungen der Gewebebehandlung im Wesentlichen elastisch verformbar ist.

2. Verband (100) nach Anspruch 1, wobei ein Mittel zum Verbinden der Polyurethanschicht und der absorbierenden Schicht (160) durch gemeinsames Zuführen der Polyurethanschicht und der absorbierenden Schicht (160) durch eine Nadelwebmaschine erhältlich ist.

3. Verband (100) nach Anspruch 1 oder Anspruch 2, wobei die absorbierende Schicht (160) umfasst:
(i) von etwa 45 % bis etwa 90 % Celluloseether-Gelierfasern; und
(ii) von etwa 10 % bis etwa 55 % Verstärkungsfasern.

4. Verband (100) nach Anspruch 1 oder Anspruch 2, wobei die absorbierende Schicht (160) Celluloseether-Gelierfasern umfasst, und wobei weiter die Polyurethanschicht mit der absorbierenden Schicht (160) durch Verstärkungsfasern gekoppelt ist, die sowohl durch die absorbierende Schicht (160) als auch durch die Polyurethanschicht hindurch genadelt sind.

5. Verband (100) nach Anspruch 3 oder Anspruch 4, wobei die Celluloseether-Gelierfasern aus mindestens einer bestehen von Carboxymethylcellulose, Carboxylethylcellulose, Hydroxyethylcellulose, Hydroxypropylcellulose und Hydroxypropylmethylcellulose.

6. Verband (100) nach einem der Ansprüche 3 bis 5, wobei die Verstärkungsfasern aus nicht gelierender Cellulose, einem Polyurethangel, einem Amidpolymer, einem Olefinpolymer, einem Esterpolymer, einem modifizierten Acrylamidpolymer oder einer Kombination oder einem Copolymer davon zusammengesetzt sind.

7. Verband (100) nach einem der Ansprüche 3 bis 6, wobei die absorbierende Schicht (160) weiter etwa 10 % bis etwa 40 % eines Alginats, Chitosans, Chitins, eines Guargummis, Pektins, eines Stärkederivats, eines Cellulosederivats, eines Glycosaminoglycans, eines Galactomannans, eines Chondroitinsalzes, Heparins, Kollagens, einer oxidierten regenerierten Cellulose (ORC), eines Heparinsalzes, Hyaluronsäure, eines Hyaluronsäuresalzes oder eine Kombination davon umfasst.

8. Verband (100) nach Anspruch 1, wobei sich die haftende Schicht (130) weiter über mindestens einen Rand der Verbundstoff-Insel erstreckt, wodurch die haftende Schicht (130) am Rand der Verbundstoff-Insel befestigt wird.

9. Verband (100) nach einem der Ansprüche 1-8, weiter umfassend eine nicht haftende Schicht, die auf der Verbundstoff-Insel, aber nicht auf der haftenden Schicht (130) angeordnet ist, wobei die nicht haftende Schicht optional perforiert ist und ein Ethylen-Methylacrylat-Copolymer umfasst.

10. Verband (100) nach einem der Ansprüche 1-9, wobei die Trägerschicht (120) feuchtigkeitsdampfdurchlässig und flüssigkeitsundurchlässig ist und ein Polyurethan umfasst.

11. Verband (100) nach einem der Ansprüche 1-10, wobei die haftende Schicht (130) ein Hydrokolloid oder einen Acrylkleber umfasst.

12. Verband (100) nach einem der Ansprüche 1-11, wobei die Polyurethanschicht eine schmelzgeblasene Polyurethanfolie, eine thermoplastische Polyurethanfolie oder ein Polyurethanschaum ist.

13. Verband (100) nach einem der Ansprüche 1-8 und 10-12, weiter umfassend eine perforierte Silikonkontaktschicht (145), die sich über mindestens den Trägerschichtrand (125) erstreckt, sich optional über den Trägerschichtrand (125) und die Verbundstoff-Insel erstreckt.

14. Verband (100) nach Anspruch 13, wobei die perforierte Silikonkontaktschicht (145) mit der haftenden Schicht (130) gekoppelt ist.

15. Verband (100) nach einem der Ansprüche 13-14, wobei die perforierte Silikonkontaktschicht (145) Perforationen (112, 115) von mindestens zwei Größen umfasst, wobei optional die Perforationen (112, 115) der Silikonkontaktschicht über der Verbundstoff-Insel kleiner sind als die Perforationen (112, 115) der Silikonkontaktschicht über dem Trägerschichtrand (125).

16. Verband (100) nach einem der Ansprüche 13-15, wobei die Perforationen (112, 115) der Silikonkontaktschicht in einer Richtung verlängert sind, um eine größere Ausdehnung des Verbandes in dieser Richtung zu ermöglichen.

## Revendications

1. Pansement (100) pour traiter un site tissulaire, le pansement comprenant :
un îlot composite comprenant une couche de polyuréthane couplée à une couche absorbante (160) comprenant des fibres gélifiantes non-tissées, dans lequel une surface de la couche absorbante (160) non couplée à la couche de polyuréthane est orientée pour être une couche inférieure ; et
une couche de support (120) s'étendant au-delà de la couche absorbante (160) pour définir une marge de couche de support (125) et une couche adhérente (130) disposée sur au moins une marge de couche de support (125),
dans lequel :
la couche de polyuréthane est sensiblement élastiquement déformable dans des conditions de traitement de tissu,
la couche absorbante (160) n'est pas élastiquement déformable dans des conditions de traitement de tissu, et
l'îlot composite est sensiblement élastiquement déformable dans des conditions de traitement de tissu.

2. Pansement (100) selon la revendication 1, dans lequel un moyen de couplage de la couche de polyuréthane et de la couche absorbante (160) peut être obtenu par coalimentation de la couche de polyuréthane et de la couche absorbante (160) à travers un métier à tisser à aiguilles.

3. Pansement (100) selon la revendication 1 ou la revendication 2, dans lequel la couche absorbante (160) comprend :
(i) environ 45 % à environ 90 % de fibres gélifiantes d'éther de cellulose ; et
(ii) environ 10 % à environ 55 % de fibres de renforcement.

4. Pansement (100) selon la revendication 1 ou la revendication 2, dans lequel la couche absorbante (160) comprend des fibres gélifiantes d'éther de cellulose, et en outre dans lequel la couche de polyuréthane est couplée à la couche absorbante (160) par des fibres de renforcement piquées à travers à la fois la couche absorbante (160) et la couche de polyuréthane.

5. Pansement (100) selon la revendication 3 ou la revendication 4, dans lequel les fibres gélifiantes d'éther de cellulose sont composées d'au moins l'une de la carboxyméthyl cellulose, la carboxyéthyl cellulose, l'hydroxyéthyl cellulose, l'hydroxypropyl cellulose, et l'hydroxypropylméthyl cellulose.

6. Pansement (100) selon l'une quelconque des revendications 3-5, dans lequel les fibres de renforcement sont composées de cellulose non gélifiante, d'un gel de polyuréthane, d'un polymère d'amide, d'un polymère d'oléfine, d'un polymère d'ester, d'un polymère d'acrylamide modifié, ou d'une combinaison ou d'un copolymère de ceux-ci.

7. Pansement (100) selon l'une quelconque des revendications 3-6, dans lequel la couche absorbante (160) comprend en outre environ 10 % à environ 40 % d'un alginate, d'un chitosane, d'une chitine, d'une gomme de guar, d'une pectine, d'un dérivé de l'amidon, d'un dérivé de la cellulose, d'un glycosaminoglycane, d'un galactomannane, d'un sel de chondroïtine, d'une héparine, d'un collagène, d'une cellulose régénérée oxydée (ORC), d'un sel d'héparine, d'un acide hyaluronique, d'un sel d'acide hyaluronique ou d'une combinaison de ceux-ci.

8. Pansement (100) selon la revendication 1, dans lequel la couche adhérente (130) s'étend en outre sur au moins une marge de l'îlot composite, fixant ainsi la couche adhérente (130) à la marge de l'îlot composite.

9. Pansement (100) selon l'une quelconque des revendications 1-8, comprenant en outre une couche non adhérente disposée sur l'îlot composite mais pas sur la couche adhérente (130), facultativement dans lequel la couche non adhérente est perforée et comprend un copolymère d'éthylène-acrylate de méthyle.

10. Pansement (100) selon l'une quelconque des revendications 1-9, dans lequel la couche de support (120) est perméable à la vapeur d'eau, imperméable aux liquides, et comprend un polyuréthane.

11. Pansement (100) selon l'une quelconque des revendications 1-10, dans lequel la couche adhérente (130) comprend un hydrocolloïde ou un adhésif acrylique.

12. Pansement (100) selon l'une quelconque des revendications 1-11, dans lequel la couche de polyuréthane est une feuille de polyuréthane de fusion-soufflage, une feuille de polyuréthane thermoplastique, ou une mousse de polyuréthane.

13. Pansement (100) selon l'une quelconque des revendications 1-8 et 10-12, comprenant en outre une couche de contact de silicone perforée (145) s'étendant sur au moins la marge de couche de support (125), s'étendant facultativement sur la marge de couche de support (125) et l'îlot composite.

14. Pansement (100) selon la revendication 13, dans lequel la couche de contact de silicone perforée (145) est couplée à la couche adhérente (130).

15. Pansement (100) selon l'une quelconque des revendications 13-14, dans lequel la couche de contact de silicone perforée (145) comprend des perforations (112, 115) d'au moins deux tailles, facultativement dans lequel les perforations de la couche de contact de silicone (112, 115) sur l'îlot composite sont plus petites que les perforations de la couche de contact de silicone (112, 115) sur la marge de couche de support (125).

16. Pansement (100) selon l'une quelconque des revendications 13-15, dans lequel les perforations de la couche de contact de silicone (112, 115) sont allongées dans une direction pour permettre une plus grande extension du pansement dans ladite direction.
